# EUROPEAN PATENT SPECIFICATION

(11) **EP 0 696 925 B1**
(45) Date of publication and mention of the grant of the patent: **20.05.1998**
(21) Application number: 94911286.6
(22) Date of filing: 05.04.1994
(51) Int. Cl.: A61M 16/10

(54) **HEATED DEVICE**
BEHEIZTE VORRICHTUNG
DISPOSITIF CHAUFFE

(30) Priority: 06.05.1993 GB 9309294
(43) Date of publication of application: 21.02.1996
(73) Proprietor: Smiths Industries Public Limited Company, London, NW11 8DS (GB)
(72) Inventor: TURNER, Mark, Folkestone, Kent CT20 2RT (GB)
(74) Representative: Flint, Jonathan McNeill
(86) International application number: GB9400722
(87) International publication number: WO9426339

(56) References cited:
- EP-A- 0 201 985
- WO-A-91/19527
- WO-A-92/07601
- GB-A- 2 233 904
- LU-A- 64 737
- US-A- 4 121 583

## Description

### Technical Field

This invention relates to HMEs or filter devices through which gas flows in a breathing gas circuit and including a heater having an electrical resistance heating element.

### Background Art

Patient breathing circuits can include heat and moisture exchange devices (HMEs). These HMEs include an element of foam or treated paper through which passes both the inhaled and exhaled gas from the patient. The exhaled gas gives up a part of its heat and moisture to the exchange element, which is then transferred to inhaled gas when the patient inhales. In this way, the gas supplied to the patient is heated and moistened reducing the risk that the patient will have a sore throat following surgery. An example of an HME is described in GB 2233904. One problem with HMEs is that condensation in the housing can lead to a build-up of water. When the problem is severe it can require the use of a hydrophobic membrane to prevent the transmission of liquid to the patient. It is undesirable to use such a membrane because it impedes gas flow through the device. To overcome this resistance to gas flow it is necessary to increase the size of the device, making it more cumbersome, heavier and more expensive. Similar problems of condensation exist in bacterial filters and the like.

One way of reducing the build up of water is to include a heater within the device. In WO 91/19527 there is described an HME including a heater located adjacent the exchange element. This reduces condensation and also improves the performance of the HME by increasing the temperature and humidity of the inhaled gas. Because, however, the heater is located centrally, it has a relatively low effect on heating the wall of the housing, which is the main area of cool surface on which condensation occurs.

### Disclosure of Invention

It is an object of the present invention to provide an improved heater or an improved heated device.

According to the present invention there is provided a heater of the above-specified kind, characterised in that the heater is separable from the device and has a surface adapted to be in thermal contact with the external surface of a wall of the device enclosing an HME or filter element such that the internal surface of the wall of the device can be heated to reduce condensation within the device.

The device and the heater may be of frusto-conical shape. The heater may have an inwardly-extending flange at one end with a central aperture, the device having a male coupling that projects through the aperture and the heater being retained on the device by means of a cooperating female coupling. The heating element may be a helical wire. The heater may include a layer of insulating material on an outside surface.

### Brief Description of Drawings

A patient breathing assembly including a filter device and a heater in accordance with the present invention will now be described, by way of example, with reference to the accompanying drawing, in which:

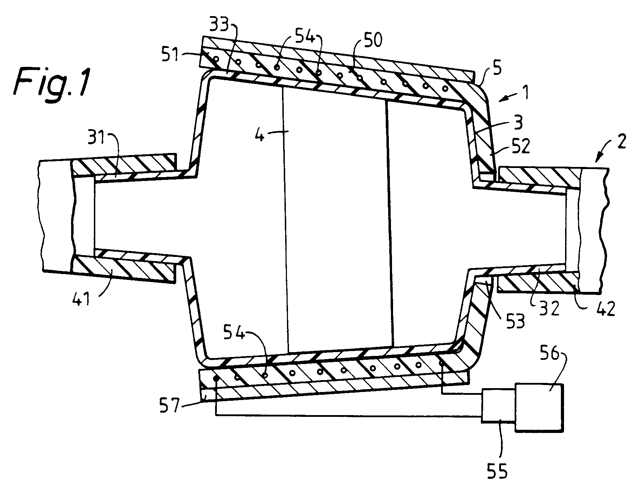

- Figure 1: is a partly sectional side elevation of the assembly; and

### Best Modes for Carrying Out the Invention

The assembly includes a filter device 1 and a patient breathing circuit, indicated generally by the numeral 2, in which the filter is connected. The device 1 has a outer housing 3 containing a conventional bacterial filter element 4 through which passes gas flowing along the breathing circuit.

The housing 3 is of a transparent plastics material such as polycarbonate and is of circular section. Two male luer taper couplings 31 and 32 are formed at opposite ends of the housing 3; these connect to female couplings 41 and 42 in the breathing circuit. The central region 33 of the housing is enlarged in diameter, compared with the couplings, and has a slight taper so that it is of frusto-conical shape.

The assembly is completed by a heater 5 closely embracing the central region 33 of the housing. The heater 5 is in the form of a frusto-conical shell 50 open at its larger, left-hand end 51 and having an inwardly-extending flange 52 lying against the right-hand side of the central region 33 of the housing. A central aperture 53 in the flange 52 is large enough to receive the right-hand luer coupling 32. The shell 50 is a close, slip fit onto the outside of the housing 3 so as to ensure a close thermal contact between the heater 5 and the housing. The heater 5 is retained on the filter device 1 by contact of the flange 52 with the female coupling 42 on the breathing circuit. The shell 50 is of a heat-resistant plastics material and includes an electrical resistance heating element 54 such as a fine metal wire extending helically around the shell. The heating element 54 may be on the inside or outside of the shell, or, as shown, incorporated within the thickness of the shell. A cable 55 connects the heating element 54 to a remote source 56 of low-power electricity, such as a battery. On the outside of the heater 5 there is a layer 57 of soft insulating material. This serves to maintain heat within the device and also provides a comfortable surface if the heater should come into contact with the patient's skin.

The heater 5 can be removed from the filter device 1 and reused on another device. It does not come into direct contact with gas flowing along the breathing circuit so it is not a source of contamination.

### Industrial Applicability

In operation, the entire interior heating surface of the heater 5 is in contact with the wall of the housing 3 of the filter device 1. The heat will be conducted through the wall of the housing to warm the inside surface. This, therefore, greatly reduces the area of cold surface in the device on which condensation can occur and, thereby, reduces the formation of liquid water. Because there is little risk of water entering the patient breathing system from the filter device, there is no need to employ a hydrophobic membrane. This avoids the need to increase the size of the device in order to compensate for the resistance to gas flow caused by such a membrane.

Instead of a filter element, the device could include a conventional heat and moisture exchange element. The heater is particularly advantageous in such applications because the increased temperature improves the performance of the HME.

Various other forms of heating element are possible. For example, a heating tape or thin-film could be used. The heater may include a positive temperature coefficient resistance so that the temperature of the heater is self regulating. The heater could extend over the couplings at opposite ends of the device to reduce condensation in this area.

## Claims

1. An HME or filter device (1,) through which gas flows in a breathing gas circuit (2) and including a heater having an electrical resistance heating element (54,), characterised in that the heater (5) is separable from the device (1,) and has a surface adapted to be in thermal contact with the external surface of a wall (3,) of the device enclosing an HME or filter element (4,) such that the internal surface of the wall of the device can be heated to reduce condensation within the device.

2. A device according to Claim 1, characterised in that the device (1,) and the heater (5) are of frusto-conical shape.

3. A device according to Claim 1 or 2, characterised in that the heater (5) has an inwardly-extending flange (52) at one end with a central aperture (53), that the device has a male coupling (32) that projects through the aperture (53), and that the heater (5) is retained on the device by means of a cooperating female coupling (42).

4. A device according to any one of the preceding claims, wherein the heating element is a helical wire (54,).

5. A device according to any one of the preceding claims, characterised in that the heater (5) includes a layer of an insulating material (57) on an outside surface.

## Patentansprüche

1. Vorrichtung zum Austausch von Wärme und Feuchtigkeit oder Filtervorrichtung (1), welche innerhalb eines Atemgaskreislaufes (2) von Gas durchströmt wird und eine Heizeinrichtung mit einem elektrischen Widerstandsheizelement (54) aufweist, **dadurch gekennzeichnet**, daß die Heizeinrichtung (5) von der Vorrichtung (1) trennbar ist und eine Oberfläche aufweist, die in thermischen Kontakt mit der äußeren Oberfläche einer Wandung (3) der eine Austauschvorrichtung für Wärme und Feuchtigkeit oder ein Filterelement (4) aufnehmenden Vorrichtung bringbar ist, wodurch die innere Oberfläche der Wandung der Vorrichtung zur Verminderung von Kondensation innerhalb der Vorrichtung beheizbar ist.

2. Vorrichtung nach Anspruch 1, **dadurch gekennzeichnet**, daß die Vorrichtung (1) und die Heizeinrichtung (5) kegelstumpfförmig ausgebildet sind.

3. Vorrichtung nach einem der Ansprüche 1 oder 2, **dadurch gekennzeichnet**, daß die Heizeinrichtung (5) an einem Ende einen sich nach innen erstreckenden Flansch (52) mit einer zentralen Öffnung (53) aufweist, die Vorrichtung eine steckerartige Kupplung (32) aufweist, welche durch die Öffnung (53) verläuft und die Heizeinrichtung (5) mit Hilfe einer entsprechenden buchsenartigen Kupplung (42) auf der Vorrichtung gehalten wird.

4. Vorrichtung nach einem der voranstehenden Ansprüche, wobei das Heizelement ein schraubenlinienförmig verlaufender Draht (54) ist.

5. Vorrichtung nach einem der voranstehenden Ansprüche, **dadurch gekennzeichnet**, daß die Heizeinrichtung (5) eine Schicht aus isolierendem Material (57) auf einer äußeren Oberfläche aufweist.

## Revendications

1. Dispositif de filtrage (1) ou HME, à travers lequel du gaz circule dans un circuit de gaz respiratoire (2) et comprenant un chauffage comportant un élément chauffant à résistance électrique (54) caractérisé en ce que le chauffage (5) est séparable du dispositif (1) et comporte une surface agencée pour être en contact thermique avec la surface externe d'une paroi (3) du dispositif renfermant un grand HME ou élément de filtrage (4) tel que la surface interne de la paroi du dispositif peut être chauffée pour réduire la condensation à l'intérieur du dispositif.

2. Dispositif selon la revendication 1, caractérisé en ce que le dispositif (1) et le chauffage (5) sont de forme tronconique.

3. Dispositif selon les revendications 1 ou 2, caractérisé en ce que le chauffage (5) comporte un rebord s'étendant vers l'intérieur (52) à une extrémité avec une ouverture centrale (53), en ce que le dispositif comprend un accouplement mâle (32) qui se projette à travers l'ouverture (53), et en ce que le chauffage (5) est retenu sur le dispositif au moyen d'un accouplement femelle coopérant (42).

4. Dispositif selon l'une quelconque des revendications précédentes, dans lequel l'élément chauffant est un fil hélicoïdal (54).

5. Dispositif selon l'une quelconque des revendications précédentes, caractérisé en ce que le chauffage (5) comprend une couche d'un matériau isolant (57) sur une surface extérieure.
